# FASCICULE DE BREVET EUROPEEN

(11) **EP 2 424 522 B1**
(45) Date de publication et mention de la délivrance du brevet: **27.07.2016**
(21) Numéro de dépôt: 10720421.6
(22) Date de dépôt: 20.05.2010
(51) Int. Cl.: A61K 31/245, A61K 31/60, A61K 33/00, A61P 9/00, A61P 9/10, A61P 25/00, A61P 23/00, A61P 43/00, A61K 9/00, A61K 31/695

(54) **COMPOSITION POUR L'ELIMINATION DE LA PLAQUE D'ATHEROME**
ZUSAMMENSETZUNG ZUR ENTFERNUNG VON ATHEROMATÖSEM PLAQUE
COMPOSITION FOR REMOVING ATHEROMATOUS PLAQUE

(43) Date de publication de la demande: 07.03.2012
(73) Titulaire: Guenoun, Gilbert, 75008 Paris (FR)
(72) Inventeur: Guenoun, Gilbert, 75008 Paris (FR)
(74) Mandataire: Chaillot, Geneviève
(86) Numéro de dépôt international: PCT/EP2010/057005
(87) Numéro de publication internationale: WO 2011/144248

(56) Documents cités:
- EP-A1- 0 391 769
- FR-A1- 2 683 455
- US-A- 3 337 406
- ASLAN A ET AL: "Action of procaine (substance H3) in the course of experimental atherosclerosis in rabbits", INFORMATION MEDICALE ROUMAINE,, 1 avril 1959 (1959-04-01), pages 92-94, XP009122435, ISSN: 0443-1251
- DATABASE CA [Online] CHEMICAL ABSTRACTS SERVICE, COLUMBUS, OHIO, US; LOEPER, J. ET AL: "The physiological role of the silicon and its anti-atheromatous action", XP002611827, extrait de STN Database accession no. 1979:149855 & LOEPER, J. ET AL: "The physiological role of the silicon and its anti-atheromatous action", NOBEL SYMPOSIUM , VOLUME DATE 1977, 40(BIOCHEM. SILICON RELAT. PROBL.), 281-96 CODEN: NOSYBW; ISSN: 0346-8313, 1978,
- DATABASE CA [Online] CHEMICAL ABSTRACTS SERVICE, COLUMBUS, OHIO, US; LOEPER, JACQUES ET AL: "Effect of silicon compounds on experimental atheroma", XP002611828, extrait de STN Database accession no. 1968:58068 & LOEPER, JACQUES ET AL: "Effect of silicon compounds on experimental atheroma", 1967, COMPTES RENDUS DES SEANCES DE LA SOCIETE DE BIOLOGIE ET DE SES FILIALES , 161(3), 589-91 CODEN: CRSBAW; ISSN: 0037-9026
- sedifa-laboratoire: "Conjonctyl", , 22 avril 2005 (2005-04-22), pages 1-4, XP002611826, Extrait de l'Internet: URL:http://web.archive.org/web/20040807124 213/sedifa-laboratoire.com/download/CONJON CTYL_POSTER_fr_A4.pdf [extrait le 2010-11-26]

## Description

L'invention concerne une nouvelle composition à caractère pharmaceutique utilisable pour l' élimination de la plaque d'athérome.

EP0391769 A1 décrit un produit thérapeutique à base d'un dérivé organique du silicium associé à une polyamine polycarboxylée, particulièrement utile pour le traitement de l'athérome.

Dans « Action de la procaïne (substance H3) au cours de l'athérosclérose expérimentale chez les lapin » (Action of procaine (substance H3) in the course of experimental atherosclerosis in rabbits »), Information médicale roumaine, 1^{er} Avril 1959, pages 92-94, Aslan A. et al. décrivent l'action de la procaïne pour freiner la formation de plaques d'athérome chez le lapin.

Dans « Effet des composés du silicium sur l'athérome expérimental » (Effect of silicon compounds on experimental atheroma), Comptes-rendus des séances de la Société de Biologie et de ses filiales, 1967, 161(3), 589-91, Jacques Loeper et al. décrivent l'effet préventif du salicylate de monométhyltrisilanol sur la formation de la plaque d'athérome, notamment par une action protectrice du développement de fibriles élastiques.

Ces documents ne divulguent pas de composition destinée à l'élimination de la plaque d'athérome.

L'invention met en oeuvre un mélange de deux principes actifs :
- de la procaïne de formule : 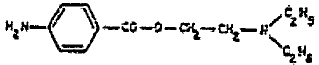 et le salicylate de monométhyltrisilanol de formule : 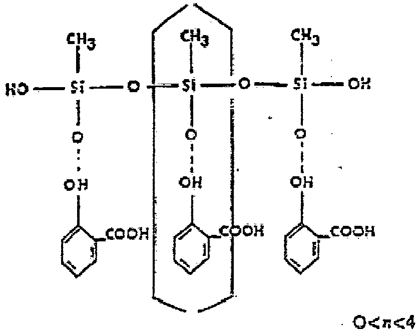 dans laquelle les liaisons hydrogènes qui unissent les fonctions OH des silanols aux fonctions phénol de l'acide salicylique sont suffisamment fortes pour empêcher, même à haute température, la polycondensation et donc l'insolubilisation du salicylate de monométhyltrisilanol (principe actif d'un médicament commercialisé sous la marque « CONJONCTYL »).

Pris individuellement ces principes actifs de médicaments sont bien connus. La procaïne a souvent été utilisée comme anesthésique local. Son utilisation en gérontologie a été envisagée. De même, le salicylate de monométhyltrisilanol constitue le principe actif d'un médicament qui, notamment par voie intraveineuse ou orale, permet une régénération du tissu conjonctif et, plus particulièrement de l'élastine et du collagène lorsqu'ils ont été détériorés sous l'effet de causes pathologiques.

Son utilisation a été recommandée pour le traitement des troubles de l'état général, plus particulièrement dans le domaine rhumatologique (arthroses, rhumatismes inflammatoires et dégénératifs, scléroses des tissus conjonctifs). De même conduit-il, dans un certain nombre de cas, à un meilleur contrôle du taux sanguin de cholestérol, des lipides et des triglycérides, d'où l'application qui en a été envisagées en cardiologie.

L'invention résulte donc de la découverte que l'administration du salicylate monométhyltrisilanol par voie intramusculaire, en présence de procaïne, permettrait d'éliminer les plaques d'athérome dans les artères correspondantes.

De préférence, les proportions des deux principes actifs au sein de la composition administrable par voie intramusculaire, varient de 30 à 60 ml de salicylate de monométhylytisilanol pour 3 à 10 ml de procaïne.

Il appartient au praticien de choisir les sites, le nombre et la fréquence des injections intramusculaires. La fréquence des injections s'établira en cures de 20 à 40 séances selon l'importance de la pathologie à traiter.

A titre d'exemple, une proportion galénique appropriée est une solution à 1% de monométhyltrisilanol orthohydroxybenzoate de sodium (4,5 ml), une solution à 1% de procaïne (0,5 ml) et de l'eau pour soluté injectable (q.s.p.) (100 ml).

Il va de soi que les deux principes actifs peuvent être utilisés sous des formes différentes, par exemple sous forme de sel pharmaceutiquement acceptables.

## Revendications

1. Composition pharmaceutique pour utilisation dans l'élimination de la plaque d'athérome contenant, en association, de la procaïne, de formule : 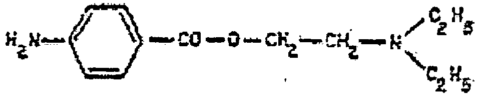 et le salicylate de monométhyltrisilanol de formule :

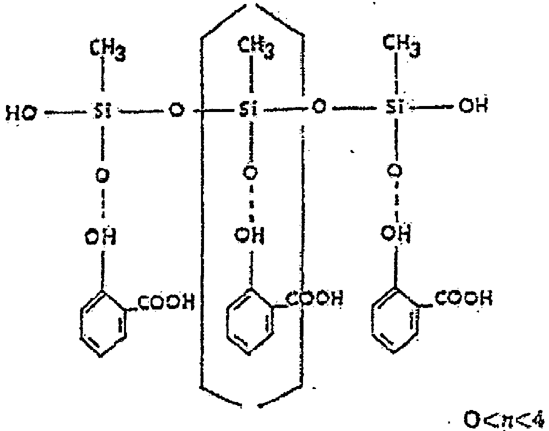

2. Composition pour utilisation selon la revendication 1, **caractérisée en ce qu'**elle comprend 30 ml de salicylate de monométhyltrisilanol pour 3 ml de procaïne.

3. Composition pour utilisation selon la revendication 1, **caractérisée en ce qu'**elle comprend 60 ml de salicylate de monométhyltrisilanol pour 10 ml de procaïne.

4. Composition pour utilisation selon l'une quelconque des revendications 1 à 3, **caractérisée en ce qu'**elle se trouve sous une forme permettant son administration par voie intramusculaire.

5. Utilisation de la composition selon l'une quelconque des revendications 1 à 4, pour la production de médicaments utilisables pour l'élimination de la plaque d'athérome.

## Patentansprüche

1. Pharmazeutische Zusammensetzung zur Entfernung von atheromatösem Plaque, die, in Kombination, Procain enthält, mit folgender Formel: 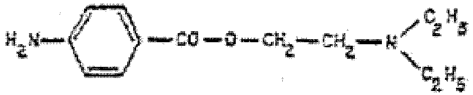 sowie Monomethyltrisilanol-Salizylat mit folgender Formel :

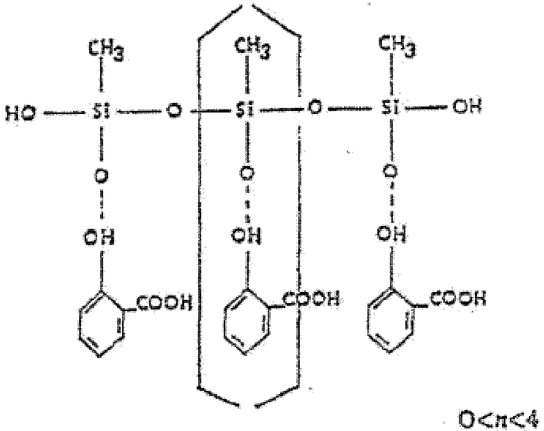

2. Zusammensetzung zur Verwendung nach Anspruch 1, **dadurch gekennzeichnet dass** sie 30 ml Monomethyltrisilanol-Salizylat pro 3 ml Procain umfasst.

3. Zusammensetzung zur Verwendung nach Anspruch 1, **dadurch gekennzeichnet dass** sie 60 ml Monomethyltrisilanol-Salizylat pro 10 ml Procain umfasst.

4. Zusammensetzung zur Verwendung nach einem beliebigen der Patentansprüche 1 bis 3, **dadurch gekennzeichnet, dass** sie sich in einer Form befindet, die ihre Verabreichung auf intramuskulärem Weg ermöglicht.

5. Verwendung der Zusammensetzung nach einem beliebigen der Patentansprüche 1 bis 4 zur Herstellung von Medikamenten, die für die Entfernung von atheromatösem Plaque verwendbar sind.

## Claims

1. A pharmaceutical composition for use in the removal of the atheromatous plaque containing, in association with each other, procaine of the formula: 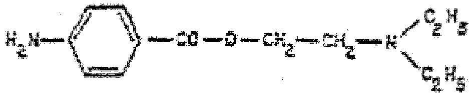 and monomethyltrisilanol salicylate of the formula:

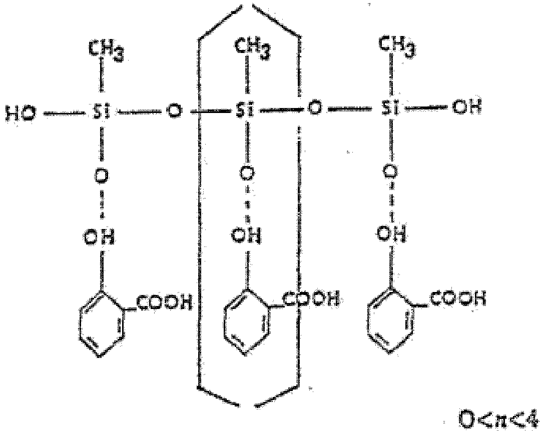

2. The composition for use according to claim 1, **characterized in that** it comprises 30 ml of monomethyltrisilanol salicylate per 3 ml of procaine.

3. The composition for use according to claim 1, **characterized in that** it comprises 60 ml of monomethyltrisilanol salicylate per 10 ml of procaine.

4. The composition for use according to any one of claims 1 to 3, **characterized in that** it is under a form which allows its administration by intramuscular route.

5. A use of the composition according to any one of claims 1 to 4 for producing drugs capable of being used for the removal of the atheromatous plaque.
